(19) Europäisches Patentamt
European Patent Office
Office européen des brevets

(11) EP 2 123 760 A1

(12) EUROPÄISCHE PATENTANMELDUNG

(43) Veröffentlichungstag:
**25.11.2009 Patentblatt 2009/48**

(51) Int Cl.:
***C12N 15/53*** *(2006.01)* ***C12N 9/04*** *(2006.01)*
***C12N 1/21*** *(2006.01)* ***C12P 7/02*** *(2006.01)*

(21) Anmeldenummer: **09010760.8**

(22) Anmeldetag: **15.04.2002**

(84) Benannte Vertragsstaaten:
**AT BE CH CY DE DK ES FI FR GB GR IE IT LI LU MC NL PT SE TR**

(30) Priorität: **20.04.2001 DE 10119274**

(62) Dokumentnummer(n) der früheren Anmeldung(en) nach Art. 76 EPÜ:
**02737953.6 / 1 383 899**

(71) Anmelder: **IEP GmbH**
**65203 Wiesbaden (DE)**

(72) Erfinder:
- **Gupta, Antje**
  **65207 Wiesbaden (DE)**
- **Breese, Klaus**
  **79115 Freiburg (DE)**
- **Bange, Gert**
  **06108 Halle (DE)**
- **Neubauer, Peter**
  **13355 Berlin (DE)**

(74) Vertreter: **Kopecky & Schwarz**
**Patentanwälte**
**Wipplingerstraße 30**
**A-1010 Wien (DE)**

Bemerkungen:
Diese Anmeldung ist am 21-08-2009 als Teilanmeldung zu der unter INID-Code 62 erwähnten Anmeldung eingereicht worden.

# (54) Enzymatisches Verfahren zur enantioselektiven Reduktion von Ketoverbindungen

(57) Die vorliegende Erfindung betrifft ein enzymatisches Verfahren zur enantioselektiven Reduktion von organischen Ketoverbindungen zu den entsprechenden chiralen Hydroxyverbindungen, eine Alkohol-Dehydrogenase aus Lactobacillus minor und ein Verfahren zur enantioselektiven Gewinnung von (S)-Hydroxyverbindung aus einem Razemat.

EP 2 123 760 A1

## Beschreibung

Die vorliegende Erfindung betrifft ein enzymatisches Verfahren zur enantioselektiven Reduktion von organischen Ketoverbindungen zu den entsprechenden chiralen Hydroxyverbindungen, eine Alkohol-Dehydrogenase aus Lactobacillus minor und ein enzymatisches Verfahren zur enantioselektiven Gewinnung von (S)-Hydroxyverbindung aus einem Razemat.

Optisch aktive Hydroxyverbindungen sind wertvolle Synthesebausteine zur Herstellung einer Vielzahl pharmakologisch wichtiger Verbindungen. Diese Verbindungen sind oft schwer herstellbar durch klassische chemische Verfahren und können die für pharmakologische Anwendungen geforderte Enantiomerenreinheit nur selten erreichen. Daher werden zur Herstellung chiraler Verbindungen in der Regel biotechnologische Verfahren angewendet, wobei die stereoselektive Reaktion entweder von ganzen Mikroorganismen oder mit isolierten Enzymen durchgeführt wird.

Dabei hat sich oft der Einsatz von isolierten Enzymen als vorteilhaft erwiesen, da mit solchen in der Regel höhere Ausbeuten sowie eine höhere Enantiomerenreinheit erzielbar sind.

Dehydrogenasen und insbesondere Alkohol-Dehydrogenasen sind wertvolle Katalysatoren zur Gewinnung von chiralen Produkten durch stereoselektive Reduktion von organischen Ketoverbindungen zu den entsprechenden chiralen Alkoholen. Bekannt sind im wesentlichen entsprechende Enzyme aus Hefe, Pferdeleber oder Thermoanaerobium brockii. Diese Enzyme benötigen als Coenzym NADH (Nicotinadenindinukleotid) oder NADPH (Nicotinadenindinukleotidphosphat). Weitere bekannte Alkohol-Dehydrogenasen sind beispielsweise eine (S)-spezifische Alkohol-Dehydrogenase aus Rhodococcus erythropolis oder eine (R)-spezifische Alkohol-Dehydrogenase aus der Gattung Lactobacillus. Beide Enzymtypen haben ein breites Substratspektrum an Ketoverbindungen und weisen eine hohe Enantioselektivität auf. Die Alkohol-Dehydrogenasen aus Lactobacillus kefir (DE 40 14 573) und Lactobacillus brevis (DE 196 10 984) eignen sich insbesondere zur Gewinnung von chiralen (R)-Alkoholen.

Nachteilig für die Anwendung von Alkohol-Dehydrogenasen sind allerdings die geringe Enzymstabilität und Enzymaktivität der Alkohol-Dehydrogenasen in organischen Lösungsmitteln und die oft nur geringe Wasserlöslichkeit der zu reduzierenden Ketoverbindungen. Ferner ist ein weiterer limitierender Faktor für die Anwendung der Alkohol-Dehydrogenasen in organischen Lösungsmitteln der notwendige Einsatz von NADP oder NAD als Cofaktorbedarf, da der Cofaktor (NADP, NAD) wasserlöslich ist und in ökonomischen Verfahren regeneriert wird.

Die Erfindung bezweckt durch Modifikation der Verfahrensbedingungen die genannten Nachteile zu verbessern. Diese Aufgabe wird erfindungsgemäß gelöst durch die Verwendung eines Zwei-Phasen-Systems, enthaltend ein organisches Lösungsmittel, Alkohol-Dehydrogenase, Wasser, Cofaktor und Ketoverbindung.

Das erfindungsgemäße Verfahren weist eine hohe Standzeit auf durch die enzymstabilisierende Wirkung des Lösemittels, eine enantiomeren Reinheit von mehr als 99,9 % der hergestellten chiralen Hydroxyverbindungen und eine hohe Ausbeute bezogen auf die eingesetzte Menge der Ketoverbindung.

Das erfindungsgemäße Verfahren betrifft daher ein Verfahren zur enantioselektiven Reduktion einer Ketoverbindung der Formel I

$$R^1\text{-C(O)-}R^2 \qquad (I)$$

wobei $R^1$ und $R^2$ unabhängig voneinander gleich oder verschieden sind und für

1. Wasserstoffatom,
2. -($C_1$-$C_{20}$)-Alkyl, worin Alkyl geradkettig oder verzweigt ist,
3. -($C_2$-$C_{20}$)-Alkenyl, worin Alkenyl geradkettig oder verzweigt ist und gegebenenfalls ein, zwei, drei oder vier Doppelbindungen enthält,
4. -($C_2$-$C_{20}$)-Alkinyl, worin Alkinyl geradkettig oder verzweigt ist und gegebenenfalls ein, zwei, drei oder vier Dreifachbindungen enthält,
5. -($C_6$-$C_{14}$)-Aryl,
6. -($C_1$-$C_8$)-Alkyl-($C_6$-$C_{14}$)-Aryl, oder
7. $R^1$ und $R^2$ bilden zusammen mit dem -C(O)-Rest ein -($C_6$-$C_{14}$)-Aryl oder einen -($C_5$-$C_{14}$)-Heterocyclus,

wobei die oben unter 1. bis 7. genannten Reste unsubstituiert sind oder ein- bis dreifach substituiert sind unabhängig voneinander durch

a) -OH,
b) Halogen, wie Fluor, Chlor, Brom oder Jod,
c) $-NO_2$,
d) -C(O)-O-($C_1$-$C_{20}$)-Alkyl, worin Alkyl gerade oder verzweigt ist und unsubstituiert oder ein- bis dreifach substituiert ist durch Halogen, Hydroxyl, Amino oder Nitro, oder
e) -($C_5$-$C_{14}$)-Heterocyclus, der unsubstituiert oder ein- bis dreifach substituiert ist durch Halogen, Hydroxyl, Amino oder Nitro,

**dadurch gekennzeichnet, dass** man

a) die Verbindung der Formel I, Alkohol-Dehydrogenase, Wasser, Cofaktor und ein organisches Lösungsmittel mit einem logP von 0,5 bis 4,0 ,

b) in einem Zwei-Phasen-System inkubiert und

c) die gebildete chirale Hydroxyverbindung isoliert.

Kohlenstoffatomen im Ring. -($C_6$-$C_{14}$)-Arylreste sind beispielsweise Phenyl, Naphthyl. Unter dem Begriff Aryl werden aromatische Kohlenstoffreste verstanden mit 6 bis 14 zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter dem Begriff "-($C_1$-$C_{20}$)-Alkyl wird ein Kohlenwasserstoffrest verstanden, dessen Kohlenstoffkette geradkettig oder verzweigt ist und 1 bis 20 Kohlenstoffatome enthält.

Der Begriff "-($C_5$-$C_{14}$)-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, O und S. Beispiele für die Begriffe -($C_5$-$C_{14}$)-Heterocyclus sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolone, Oxadiazolidindione, Triazole, welche durch F, -CN, -$CF_3$ oder -C(O)-O-($C_1$-$C_4$)-Alkyl substituiert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen ableiten. Insbesondere bevorzugt sind die-Reste 2- oder 3-Pyrrolyl, Phenylpyrrolyl wie 4-oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3-oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Bevorzugte Verbindungen der Formel I sind 4-Chlor-3-oxo- butansäureethylester, Acetophenon, Acetessigsäuremethylester, Ethyl-2-oxo-4-phenylbutyrat, 2,5-Hexandion Ethylpyruvat oder 2-Octanon, bevorzugt 4-Chlor-3-oxo-butansäureethylester. Die Verbindungen der Formel I werden im erfindungsgemäßen Verfahren in einer Menge von 2 % bis 30 % bezogen auf das Gesamtvolumen eingesetzt, bevorzugt von 10 % bis 25 %, insbesondere von 15 % bis 22 %.

Dem Wasser wird bevorzugt ein Puffer zugesetzt, beispielsweise Kaliumphosphat, Tris/HCl- oder Triethanolamin-Puffer mit einem pH-Wert von 5 bis 10, vorzugsweise ein pH-Wert von 6 bis 9. Die Pufferkonzentration beträgt von 10 mM bis 150 mM, bevorzugt von 90 mM bis 110 mM, insbesondere 100 mM. Zusätzlich enthält der Puffer auch Magnesiumionen, beispielsweise $MgCl_2$ in einer Konzentration von 0,2 mM bis 10 mM, bevorzugt 0,5 bis 2 mM, insbesondere 1 mM.

Die Temperatur beträgt beispielsweise von etwa 10 °C bis 70 °C, bevorzugt von 30 °C bis 60 °C.

Die erfindungsgemäß einsetzbaren organischen Lösungsmittel haben bevorzugt einen logP von 0,6 bis 2,0, insbesondere von 0,6 bis 1,9 , insbesondere bevorzugt von 0,63 bis 1,75. Die bevorzugten organischen Lösungsmittel sind beispielsweise Diethylether, tertiär-Butylmethylether, Diisopropylether, Dibutylether oder Essigsäureethylester, insbesondere Essigsäureethylester. Essigsäureethylester kann beispielsweise in einer Menge von 1 % bis 90 % bezogen auf das Gesamtvolumen des Reaktionsansatzes eingesetzt werden, vorzugsweise von 15 % bis 60 %, insbesondere von 20 % bis 50 %.

Das Verhältnis von organischen Lösungsmittel zu Wasser beträgt von 9 zu 1 bis 1 zu 9, vorzugsweise von 1 zu 1 bis 1 zu 3.

Das Wasser bildet im erfindungsgemäßen Zwei-Phasen-System die eine flüssige Phase und das organische Lösungsmittel bildet die zweite flüssige Phase. Geigebenenfalls kann auch noch eine feste oder weitere flüssige Phase vorliegen, die beispielsweise durch nicht vollständig gelöste Alkohol-Dehydrogenase oder durch die Verbindung der Formel I entsteht. Bevorzugt sind jedoch zwei flüssige Phasen ohne feste Phase. Die zwei flüssigen Phasen werden bevorzugt mechanisch gemischt, so dass große Oberflächen zwischen den beiden flüssigen Phasen erzeugt werden.

Die Konzentration des Cofaktors NADPH oder NADH bezogen auf die wäßrige Phase beträgt von 0,05 mM bis 0,25 mM, insbesondere von 0,06 mM bis 0,2 mM.

Bevorzugt wird im erfindungsgemäßen Verfahren noch ein weiterer Stabilisator der Alkohol-Dehydrogenase eingesetzt. Geeignete Stabilisatoren sind beispielsweise Glycerin, Sorbitol oder Dimethylsulfoxid (DMSO).

Die Menge an Glycerin beträgt von 5 % bis 30 % bezogen auf das Volumen des gesamten Ansatzes. Bevorzugte Mengen an Glycerin sind von 10 % bis 20 %, insbesondere 20 %.

Zu Regenerierung des verbrauchten NADH oder NADPH kann im erfindungsgemäßen Verfahren zusätzlich Isopropanol zugefügt werden. Beispielsweise wird das Isopropanol und NADP mit der Alkohol-Dehydrogenase zu NADPH und Aceton umgesetzt. Die eingesetzte Isopropanolmenge beträgt von 5 % bis 30 % bezogen auf das Volumen des gesamten Ansatzes. Bevorzugte Mengen an Isopropanol sind von 10 % bis 20 %, insbesondere 10 %.

Geeignete Alkohol-Dehydrogenasen stammen beispielsweise aus Hefe, Pferdeleber oder Rhodococcus erythropolis, wobei diese Enzyme als Coenzym NADH benötigen, oder aus Thermoanaerobium brockii, Lactobacillus kefir oder Lactobacillus brevis, wobei diese Enzyme als Coenzym NADPH benötigen.

Wird eine Alkohol-Dehydrogenasen beispielsweise aus Hefe, Pferdeleber, Thermoanaerobium brockii oder Rhodococcus erythropolis im erfindungsgemäßen Verfahren eingesetzt, so wird aus der Verbindung der Formel I die entsprechende (S)-Hydroxyverbindung gewonnen. Wird eine Alkohol-Dehydrogenasen beispielsweise aus Lactobacillus kefir oder Lactobacillus brevis im erfindungsgemäßen Verfahren eingesetzt, so wird aus der Verbindung der Formel I die entsprechende (R)-Hydroxyverbindung gewonnen.

Die Alkohol-Dehydrogenase kann in dem erfindungsgemäßen Verfahren entweder vollständig gereinigt oder teilweise gereinigt eingesetzt werden oder in Zellen enthaltend verwendet werden. Die eingesetzten Zellen können dabei nativ, permeabilisiert oder lysiert vorliegen.

Die Volumenaktivität der eingesetzten Alkohol-Dehydrogenase beträgt von 100 Units/ml (U/ml) bis 2000 U/ml, bevorzugt etwa 800 U/ml, bei einem Proteingehalt von etwa 20 mg/ml bis 22 mg/ml. Die bevorzugt eingesetzte Alkohol-Dehydrogenase hat eine spezifische Aktivität von etwa 35 bis 40 U/mg Protein. Je kg umzusetzender Verbindung der Formel I werden 20 000 bis 200 000 U Alkohol-Dehydrogenase eingesetzt, bevorzugt etwa 100 000 U. Der Enzymeinheit 1 U entspricht dabei der Enzymmenge die benötigt wird um 1 $\mu$mol der Verbindung der Formel I je Minute (min) umzusetzen.

Das erfindungsgemäße Verfahren wird beispielsweise in einem geschlossenen Reaktionsgefäß aus Glas oder Metall durchgeführt. Dazu werden die Komponenten einzeln in das Reaktionsgefäß überführt und Rühren unter einer Atmosphäre von beispielsweise Stickstoff oder Luft gerührt. Je nach Substrat und eingesetzter Verbindung der Formel I beträgt die Reaktionszeit von 1 Tag bis 14 Tage, bevorzugt 4 bis 7 Tage.

Anschließend wird das Reaktionsgemisch aufgearbeitet. Dazu wird die wäßrige Phase abgetrennt, die Essigsäureethylester-Phase wird gefiltert. Die wäßrige Phase kann gegebenenfalls noch einmal extrahiert werden und wie die Essigsäureethylester-Phase weiter aufgearbeitet werden. Danach wird die gefilterte Phase unter vermindertem Druck verdampft. Man erhält so beispielsweise das Produkt 4-Chlor-3-(S)-hydroxy-butansäureethylester mit einer Enantiomerenreinheit von mehr als 99,9% und im wesentlichen frei vom Edukt 4-Chlor-3-oxo- butansäureethylester. Die Gesamtausbeute der Prozesse beträgt nach Destillation des Produktes von 82 % bis 88% bezogen auf die eingesetzte Eduktmenge.

Überraschenderweise zeigen die organischen Lösungsmittel mit einem log-P-Wert von 0 bis 4 eine stabilisierende Wirkung auf die Alkohol-Dehydrogenase, während im Stand der Technik von der Verwendung der Zwei-Phasen-Systeme mit organischen Lösungsmittel abgeraten wird (M.R. Kula, U. Kragel; Kapitel 28, Dehydrogenases in Synthesis of Chiral Compounds; R. N. Patel, Stereoselective Biocatalyses, 2000; Peters J. 9. Dehydrogenases-Characteristics, Design of Reaction Conditions, and Application, In: H.J. Rehm, G. Reed Biotechnology, Vol 3, Bioprocessing, VCH Weinheim, 1993; J. Lynda et al., Solvent selection strategies for extractive Biocatalysis, Biotechnol. Prog. 1991, 7, Seiten 116- 124). Im erfindungsgemäßen Verfahren wird als organische Phase Essigsäureethylester verwendet , wobei die organische Phase zum einen als Reservoir für die Verbindung der Formel I dient, aber auch gleichzeitig das Reaktionsprodukt, die chirale Hydroxyverbindung aus der wäßrigen Phase extrahiert.

Im Gegensatz zum Stand der Technik führt der Einsatz von organischen Lösungsmitteln mit einem log-P-Wert von 0 bis 3 zu einer zusätzlichen im Zeitverlauf zunehmenden Stabilisierung der Alkohol-Dehydrogenase. Im Stand der Technik üben insbesondere organische Lösungsmittel mit einem log-P-Wert(Logarithmus des Octanol/ Wasser- Verteilungskoeffizienten) von 0 bis 2 eine besonders instabilisierende Wirkung auf Enzyme aus und kommen somit als organische Phase im Zwei-Phasen-System kaum in Betracht (K. Faber, Biotransformations in organic chemistry, 3rd edition 1997, Springer Verlag, Kapitel 3. Bis 3.17).

Die Erfindung betrifft ferner die Alkohol-Dehydrogenase aus Lactobacillus minor mit hohem Temperaturoptimum. Die Alkohol-Dehydrogenase aus Lactobacillus minor hat die DNA-Sequenz gemäß SEQ ID NO: 3 und die Aminosäuresequenz gemäß SEQ ID NO: 4 gemäß des beiliegenden Sequenzprotokolls. Diese Alkohol-Dehydrogenase aus Lactobacillus minor ist R-spezifisch, wobei beispielsweise aus einer Verbindung der Formel I die entsprechende (R)-Hydroxyverbindung gewonnen werden kann. Die enantioselektive Alkohol-Dehydrogenase aus Lactobacillus minor lässt sich überraschenderweise in Escherichia coli RB 791 überexprimieren, während Alkohol-Dehydrogenasen aus anderen Arten der Gattung Lactobacillus nur in wesentlich geringerer Weise exprimiert werden konnten. Dies ist um so überraschender, da die Alkohol-Dehydrogenase im Wildstamm von Lactobacillus minor selbst nur sehr gering exprimiert wird und somit mit gängigen Screeningverfahren (Ganzzellbiotransformation, Aktivitätstest) nicht nachweisbar war. Es war daher sehr überraschend, dass sich aus Lactobacillus minor eine R-enantioselektive Alkohol-Dehydrogenase klonieren ließ und in Escherichia coli so außerordentlich stark überexprimierbar war (50% des Zellproteins des Klons, 20.000 Units/g Feuchtgewicht).

Das gereinigte Enzym aus Lactobacillus minor ist stabil in einem pH-Bereich von etwa 5,5 bis 8,5. Das Enzym ist bis etwa 40 °C stabil und das pH-Optimum der enzymatischen Reaktion liegt im Bereich von pH 7 bis pH 7,5. Das Temperaturoptimum der enzymatischen Reaktion liegt bei etwa 55 °C. das Enzym weist ein breites Substratspektrum auf.

Das Enzym läßt sich mittels hydrophober Interaktionschromatographie bis zu einer spezifischen Aktivität von 35 bis 40 U/mg Protein reinigen.

Die Erfindung betrifft auch ein Verfahren zur Gewinnung der Alkohol-Dehydrogenase aus Lactobacillus minor. Dazu wird die DNA, die für die Alkohol-Dehydrogenase aus Lactobacillus minor kodiert, in einem geeigneten prokaryotischen oder eukaryotischen Mikroorganismus exprimiert. Bevorzugt wird die Alkohol-Dehydrogenase aus Lactobacillus minor in einen Escherichia coli Stamm transformiert und exprimiert, insbesondere in Escherichia coli RB 791.

Die Alkohol-Dehydrogenase aus Lactobacillus minor läßt sich beispielsweise so gewinnen, dass die rekombinanten Escherichia coli Zellen kultiviert werden, die Expression der Alkohol-Dehydrogenase induziert wird und anschließend nach etwa 10 bis 18 Stunden (h) die Zellen durch Ultraschallbehandlung oder durch French-Press (Gaullin, Siemens) aufgeschlossen werden. Der erhaltene Zellextrakt kann entweder direkt verwendet werden oder weiter gereinigt werden. Dazu wird der Zellextrakt beispielsweise zentrifugiert und der erhaltene Überstand wird einer hydrophoben Interaktionschromatographie unterworfen. Diese Chromatographie erfolgt bevorzugt bei pH 7,0 in einem wässrigen Puffer der auch Magnesiumionen enthält.

Die Erfindung betrifft ferner ein Verfahren zur Gewinnung einer enantioselektiven (S)-Hydroxyverbindung der Formel II

$$R^1\text{-C(OH)-}R^2 \qquad \text{(II)}$$

wobei $R^1$ und $R^2$ unabhängig voneinander gleich oder verschieden sind und für

1. Wasserstoffatom,
2. -($C_1$-$C_{20}$)-Alkyl, worin Alkyl geradkettig oder verzweigt ist,
3. -($C_2$-$C_{20}$)-Alkenyl, worin Alkenyl geradkettig oder verzweigt ist und gegebenenfalls ein, zwei, drei oder vier Doppelbindungen enthält,
4. -($C_2$-$C_{20}$)-Alkinyl, worin Alkinyl geradkettig oder verzweigt ist und gegebenenfalls ein, zwei, drei oder vier Dreifachbindungen enthält,
5. -($C_6$-$C_{14}$)-Aryl,
6. -($C_1$-$C_8$)-Alkyl-($C_6$-$C_{14}$)-Aryl, oder
7. $R^1$ und $R^2$ bilden zusammen mit dem -C(O)-Rest ein -($C_6$-$C_{14}$)-Aryl oder einen -($C_6$-$C_{14}$)-Heterocyclus, wobei die oben unter 1. bis 7. genannten Reste unsubstituiert sind oder ein- bis dreifach substituiert sind unabhängig voneinander durch

   a) -OH,
   b) Halogen, wie Fluor, Chlor, Brom oder Jod,
   c) -$NO_2$,
   d) -C(O)-O-($C_1$-$C_{20}$)-Alkyl, worin Alkyl gerade oder verzweigt ist und unsubstituiert oder ein- bis dreifach substituiert ist durch Halogen, Hydroxyl, Amino oder Nitro, oder
   e) -($C_6$-$C_{14}$)-Heterocyclus, der unsubstituiert oder ein- bis dreifach substituiert ist durch Halogen, Hydroxyl, Amino oder Nitro,

**dadurch gekennzeichnet, dass** man

a) ein racemisches Gemisch, enthaltend die Verbindung der Formel II, die erfindungsgemäße Alkohol-Dehydrogenase, Wasser, Cofaktor und ein organisches Lösungsmittel ein organisches Lösungsmittel mit einem logP von 0,5 bis 4,0, beispielsweise aus der Reihe Diethylether, tertiär-Butylmethylether,
Diisopropylether oder Essigsäureethylester,

b) in einem Zwei-Phasen-System inkubiert und

c) die gebildete enantiomerenreine (S)-Hydroxyverbindung isoliert.

Die Reaktionsbedingungen sind im wesentlichen dieselben wie im obengenannten Verfahren zur enantiospezifischen Reduktion der Ketoverbindung der Formel I. Im Verfahren wird jedoch statt einer enantioselektiven Reduktion der Ketoverbindung der Formel I, die ensprechende (R)-Hydroxyverbindung der Formel II zur entsprechenden Ketoverbindung oxydiert. Ferner wird im Verfahren anstelle von Isopropanol Aceton zur Regenerierung von NADP eingesetzt. Beispielsweise wird das Aceton und NADPH mit der erfindungsgemäßen Alkohol-Dehydrogenase zu NADP und Isopropanol umgesetzt. Die eingesetzte Acetonmenge beträgt von 5 % bis 30 % bezogen auf das Volumen des gesamten Ansatzes. Bevorzugte Mengen an Aceton sind von 10 % bis 20 %, insbesondere 10 %.

Die erfindungsgemäße Alkohol-Dehydrogenase kann für die Herstellung der Verbindung der Formel II entweder vollständig oder teilweise gereinigt vorliegen oder kann auch enthaltend in Zellen im Verfahren eingesetzt werden. Die Zellen können dabei nativ, permeabilisiert oder lysiert vorliegen.

Gegenstand der Erfindung ist auch ein rekombinanter Klon von Escherichia coli RB 791, der die Alkohol-Dehydrogenase aus Lactobacillus minor exprimiert und am 26. März 2001 unter den Bedingungen des Budapester Vertrages bei der Deutschen Sammlung für Mikroorganismen und Zellkulturen, Mascheroder Weg 1b, 38124 Braunschweig unter der Nummer DSM 14196 hinterlegt wurde.

Die Erfindung wird durch die folgenden Beispiele erläutert:

Beispiel 1

Screening nach R-Alkohol-Dehydrogenasen in Stämmen der Gattung Lactobacillus mittels Ganzzellbiotransformation

Zum Screening wurden verschiedene Lactobacillenstämme in folgendem Medium kultiviert (Angaben jeweils g/l): Glucose (20), Hefeextrakt (5), Fleischextrakt (10), Di-Ammoniumhydrogencitrat (2), Natriumacetat (5), Magnesiumsulfat (0,2), Mangansulfat (0,05), Di-Kaliumhydrogenphosphat (2).

Das Medium wurde bei 121 °C sterilisiert und die Stämme der Gattung Lactobacillus (im folgenden mit L. abgekürzt) wurden ohne weitere pH-Regulierung oder Sauerstoffzufuhr kultiviert. Anschließend wurden die Zellen abzentrifugiert und für die Ganzzellbiotransformation wurden jeweils 4g Zellen in einem Endvolumen von 10 ml Kaliumphosphatpuffer (KPi-Puffer) (50 mM, pH = 7.0) resuspendiert. Nach Zugabe von je 0,1 g Glucose wurden die Zellen für 15 min bei 30 °C geschüttelt. Zur Zellsuspension wurde 4-Chlor-3-oxo- butansäureethylester 4 in einer Endkonzentration von 40 mM zugegeben und nach jeweils 10 min und 120 min erfolgte die gaschromatographische Analyse des Medium. Dazu wurden die Zellen abzentrifugiert, der Überstand filtriert und in Chloroform bis zu einer Endkonzentration von 10-15 μg/ml 4-Chlor-3-oxo-butansäureethylester verdünnt.

Der als Substrat eingesetzte 4-Chlor-3-oxo-butansäureethylester wurde von den verschiedenen Lactobacillenstämmen mit folgender Enantiomerenreinheit zum Ethyl (S)-4 chloro-3-hydroxybutyrat umgesetzt.

Der Enantiomerenüberschuß berechnet sich wie folgt: ee(%) = ((R-Alkohol -S-Alkohol)/(R-Alkohol+ S-Alkohol)) x 100.

Tabelle 1

| Stamm Lactobacillus | ee 4-Chlor-3-(S)-hydroxy-butansäureethylester in % |
|---|---|
| L. reuteri | 34,6 |
| L. kandleri | 90,0 |
| L. collinoides | 71,3 |
| L. bifermentans | 53,6 |
| L. oris | 63,4 |
| L. brevis | 74,0 |
| L. halotolerans | 67,2 |
| L. minor | 18,6 |
| L. parabuchneri | 78,5 |
| L. kefir | 87,8 |
| L. fructosus | 28,9 |

Beispiel 2

Gewinnung rekombinanter R-spezifischer Alkohol-Dehydrogenasen

A.) Präparation genomischer DNA aus Stämmen der Gattung Lactobacillus

Das Zellpellet aus etwa 2 ml Kulturflüssigkeit der Gattung Lactobacillus wurde in 300 µl TE-Puffer (enthaltend 10 mM Tris/HCl, pH = 8, 1 mM EDTA) resuspendiert, mit 20 mg/ml Lysozym versetzt und 10 min bei 37°C inkubiert. Anschließend wurden 100 µl Natriumdodecylsulfat (SDS) (10%), 100µl Na-Perchlorat (5M) und 500 µl Chloroform/ Isoamylalkohol (24:1) zugegeben. Nach kräftigem Schütteln wurde das Protein abzentrifugiert und die wäßrige Phase in ein neues Eppendorfgefäß überführt. Danach wurden 800 µl Ethanol (EtOH) (96 %) zugegeben. Das Eppendorfgefäß wurde mehrfach invertiert und anschließend die ausgefallene chromosomale DNA in ein neues Eppendorfgefäß überführt und mit 200 µl. EtOH gewaschen. Die DNA wurde wiederum in ein neues Eppendorfgefäß überführt, unter verminderten Druck getrocknet und in 100µl TE-Puffer gelöst.

B.) Oligonukleotide als 5'- und 3'-Primer für die PCR (Polymerase chain reaction)

Die für die PCR verwendeten Primer wurden von der bekannten N-terminalen und C-terminalen Sequenz der Alkohol-Dehydrogenase aus L. kefir abgeleitet. Dabei wurden bekannte Präferenzen für bestimmte Codons in Lactobacillen berücksichtigt. So wurde vor jeden 5'-Primer das Codon ATG (Met) als Startcodon vorgesetzt, weiterhin wurde am 5'-Primer dem Startcodon die Schnittstelle für das Restriktionsenzym Bam HI (GGATCC) vorangestellt um die spätere Klonierung in den Expressionsektor zu ermöglichen. Hinter den 3'-Primer wurde das Stop codon (TAG) und die Schnittstelle für Hind III (AAGCTT) gesetzt. Die Primerkonstrukte sind im folgenden aufgelistet
N = A,T, C oder G; Y = T oder C; R = A oder G
5'Primer
5'GCGGATCCATGACNGAYCGNTTRAARGGNAARGTNGC3' (SEQ ID NO:1)
3'Primer
5'GGGAAGCTTCTAYTGNGCNGTRTANCCNCCRTCNAC3' (SEQ ID NO:2)
Die Primer wurden nach bekannten Verfahren hergestellt.

C.) PCR ( Polymerase chain reaction) mit der genomischen DNA aus Stämmen der Gattung Lactobacillus

PCR-Ansatz (100µl):

| | Einsatz pro Reaktion | Konzentration |
|---|---|---|
| dNTP's | 8µl | je NTP 2.5 nmol/µl |
| Oligos | je Oligo 10µl: 20µl | 2pmol/µl |
| chromosomale DNA | 3µl | ca. 1µg/µl |
| 10 x Puffer (Promega) | 10µl | |
| Taq- Polymerase (Promega) | 1µl | 2U/µl |
| $H_2O$ | 58µl | |
| dNTP's sind ein Gemisch von Desoxynucleotidtriphosphaten wie dATP, dGTP, dCTP, dTTP | | |

<u>Zyklus:</u>

95 °C 2 min, danach
80 °C halten
heißer Start, danach
95 °C 30 sec, danach

40 °C 1 min 30x
danach jeweils 30 mal 95 °C 30 sec, und 40 °C 1 min, anschließend
72 °C 2,5 min
danach
72 °C 2,5 min
danach
10 °C halten

Für die Analytik wurden 10 μl des Ansatzes auf ein 1 %iges Agarosegel aufgetragen und bei konstant 100 V elektrophoretisch aufgetrennt. Die PCR zeigte die deutliche Amplifikation eines DNA-Stückes von etwa 750 bp.

D.) Isolierung der PCR-Fragmente aus dem Gel

Zur Gewinnung des PCR-Fragments wurde der gesamte PCR-Ansatz auf ein 1 %iges Agarosegel aufgetragen und bei konstant 100V elektrophoretisch aufgetrennt. Dazu wurde das Gel in zwei Spuren geteilt, wovon eine den kompletten PCR-Ansatz und die andere nur eine Probe von 5 μl enthielt, so dass zum Ausschneiden des PCR-Fragments aus dem Gel zur Orientierung nur die Spur mit der Probe mit Ethidiumbromid angefärbt wurde um eine Schädigung des zu isolierenden PCR-Fragment durch Ethidiumbromid und durch UV-Licht auszuschließen.
Die Isolierung aus dem Gel erfolgte mit dem QIAquick Gel Extraction Kit von der Firma Qiagen aus Hilden.
Die Konzentrationsbestimmung ergab eine Gesamtkonzentration von 20 ng/μl DNA.

E.) Ligation

Zur Vorbereitung der Ligation wurden das gereinigte PCR-Fragment und der verwendete Klonierungsvektor pQE30 oder pQE 70 beide von der Firma Quiagen mit Bam HI und Hind III geschnitten (4μl DNA= 200ng DNA, 1μl 10xPuffer, 1μl Enzym, BSA und $H_2O$ (Biolabs, New England)).
Das geschnittene Plasmid wurde dann erneut mittels QIAquick Gel Extraction Kit gereinigt, in Wasser aufgenommen mittels alkalischer Phosphatase dephosphoryliert (USB, Amersham Life Science).
Zur Reinigung wurden die .entsprechenden Reaktionsansätze erneut auf ein 1%iges Agarosegel aufgetragen und so das verdaute Amplifikat sowie das Plasmid wie unter D.) beschrieben aus dem Gel isoliert. Die Konzentration von Plasmid und Amplifikat nach der Reinigung betrug etwa 20 ng/μl.
Zur Ligation wurden 3 μl pQE30 oder pQE 70 (60ng), 2,5μl Amplifikat (50ng), 2μl Ligasepuffer (Boehringer; Mannheim), 1,5 μl $H_2O$ und 1μl T4-Ligase (Boehringer; Mannheim) eingesetzt. Der Ansatz wurde über Nacht bei 16 °C inkubiert.
Anschließend wurden 40 μl elektrokompetente Zellen von Escherichia coli RB791 mit 1,5 μl Ligationsansatz durch Elektroporation transformiert. Die Zellen wurden in 500 μl SOC-Medium gegeben, 45 min bei 37 °C inkubiert und anschließend je 250 μl auf $LB_{amp}$-Agarplatten ausplattiert. Das SOC-Medium enthält pro Liter Wasser 20 g Trypton, 5 g Hefe-Extrakt, 0,5 g NaCl, 10 ml von 1 M $MgSO_4$ und 10 ml von 1 M $MgCl_2$. $LB_{amp}$-Agarplatten enthalten pro Liter Wasser 10 g Trypton, 5 g Hefe-Extrakt, 10 g NaCl, 20 g Agar, pH 7,0 und 50 mg Ampicillin.
Gewachsene Kolonien wurden abgeimpft und in 4ml Flüssigkultur ($LB_{amp}$-Medium) über Nacht bei 37 °C kultiviert. Von dieser Zellsuspension wurden je 2 ml zur Plasmidpräparation (entsprechend dem Quiagen miniprep Protokoll (Quiagen, Hilden)) eingesetzt.
Von der Plasmidpräparation wurden ein Restriktionsverdau mit Bam HI und HindIII angesetzt. Der komplette Verdau wurde auf ein 1 % iges Agarosegel aufgetragen und bei 100 V elektrophoretisch aufgetrennt (Nachweis des 750 kp Inserts) und die Plasmide daraufhin gegebenenfalls zur Sequenzierung eingesetzt.
Klone mit 750 kp Insert wurden dann auf $LB_{amp}$-Agarplatten ausplattiert.

F.) Sequenzierung der Plasmide

Die Sequenzierung wurde mittels dem SequiThermEXCEL II Long-Read DNA Sequencing Kit (Biozym, Oldendorf) am Li-Cor-Sequenzer (MWG Biotech, Ebersberg) entsprechend den Angaben des Herstellers durchgeführt. Als Primer wurden die Standard Sequenzierungsprimer für pQE-Vektoren benutzt.

G.) Screening der Klone hinsichtlich löslicher Expression der R-ADH

Klone mit Inserts von 750 kp wurden hinsichtlich enzymatischer Aktivität und Stereoselektivität untersucht. Dazu wurden die Kolonien von den $LB_{amp}$-Agarplatten abgeimpft und in 20 ml Flüssigkulturen ($LB_{amp}$-Medium) bei 25 °C kultiviert. Bei einer Zelldichte ($OD_{500}$) von 0,5 erfolgte dann die Induktion mit 1 mM Isopropyl-β-D-thiogalactopyranosid (IPTG). Nach 18 h wurden die Zellen abzentrifugiert und je 40 mg Zellen in 350 µl Kpi-Puffer (50mM, pH = 7, 1mM $MgCl_2$) aufgenommen. Die Enzymfreisetzung aus den Zellen wurde durch Naßvermahlung mit Hilfe von Glasperlen (0,5 g, 0,3 mm) erreicht. Dazu erfolgte ein 20 minütlicher Aufschluß mittels Retsch-Mühle bei 4°C.

Der Enzymtest enthielt 870 µl Triethanoiaminpuffer (100mM, pH=7,0, 1mM $MgCl_2$), 100 µl einer 100mmolaren Lösung 4-Cl-Acetessigsäureethylester, 10 µl NADPH (Endkonzentration 0,19mM) und 20 µl Enzymlösung.

Die Definition der Enzymeinheit: 1 U entspricht der Enzymmenge die benötigt wird um 1 µmol Substrat (4-Chlor-3-oxo- butansäureethylester) pro 1 min umzusetzen.

Zum Nachweis der Stereoselektivität wurden 480 µl Triethanolaminpuffer (100mM, pH=7,0, 1mM $MgCl_2$ ) mit 1,0 mM 4-Chlor-3-oxo-butansäureethylester, 1,9 mM NADPH (jeweils Endkonzentration) und 20 µl Enzymlösung inkubiert. Nach 15 min Inkubation wurde der Reaktionsansatz filtriert und 1:10 in Chloroform verdünnt und eine Probe wurde mittels GC-MS analysiert.

Bedingungen der Gaschromatographie (GC):

chirale Säule: Lipodex E, ID = 0,25mm, I = 25m (Macherey-Nagel)

1. 2 min 60°C
2. in 28 min von 60 °C auf 130 °C mit einer Rate von 2,5 °C pro Minute
3. 15 min bei 130 °C

Aus den folgenden Lactobacillenstämmen konnte eine (R)-spezifische Alkohol-Dehydogenase kloniert und aktiv überexprimiert werden:

| Stamm | Plasmid | Klon Nummer | Aktivität in U/g Zellen* | ee in % |
|---|---|---|---|---|
| L. parabuchneri | pQE 30 | 12 | 450 | >99,9 |
| L. parabuchneri | pQE 30 | 14 | 170 | >99,9 |
| L. kandleri | pQE 30 | 11 | 280 | >99,9 |
| L. kandleri | pQE 70 | 17 | 710 | >99,9 |
| L. minor | pQE 30 | 2 | 2.830 | >99,9 |
| L. minor | pQE 70 | 3 | 680 | >99,9 |
| L. minor | pQE 70 | 4 | 700 | >99,9 |
| * Aktivität berechnet aus G.) (Naßvermahlung); Die Aktivitäten liegen nach Fermentation und Aufschluß mit French-Press erheblich höher. | | | | |

H.) Enzymgewinnung und Reinigung

Der Stamm mit der höchsten enzymatischen Aktivität wurde zur Enzymgewinnung im Fermenter ( Fed batch, 10 l) kultiviert. Die Induktion erfolgte bei einer $OD_{500}$ von 40 mit 1 mM IPTG. Nach 18 h erfolgte die Zellernte wobei 300 g Zellen in 3 l Kpi-Puffer (50mM, pH = 7, 1mM $MgCl_2$) aufgenommen wurden, anschließend erfolgte der Zellaufschluß mittels French-Press (Gaullin, Siemens). Der nach Zentrifugation erhaltene Überstand wird im folgenden als Rohextrakt bezeichnet und wies eine Volumenaktivität von etwa 2000 U/ml (20 000 U/g Feuchtmasse) auf.

Für die Enzymcharakterisierung wurde ein Teil des erhaltenen Enzyms mittels hydrophober Interaktionschromatographie auf Q-Sepharose ff (fast flow) gereinigt. Die verwendete Säule wurde dazu mit 50mM Kpi-Puffer pH = 7.0, 1mM $MgCl_2$ äquilibriert. Nach Auftragen des Rohextrakts auf die Säule und kurzem Spülen mit dem Äquilibrierungspuffer wurde das Enzym mit einem steigenden linearem Salzgradienten (0-1 M NaCl, 1ml/min) bei einer Salzkonzentration von etwa 0,3 M NaCl eluiert. Nach Vereinigen der enzymhaltigen Fraktionen erhielt man etwa 25 ml des gereinigten Enzyms mit einer Volumenaktivität von etwa 800 U/ml und einem Proteingehalt von 20 bis 22 mg/ml. Das so gereinigte Enzym hat also eine spezifische Aktivität von etwa 35 bis 40 U/mg Protein.

Alle enzymatischen Aktivitäten wurden bei 25 °C bestimmt. Die Enzymaktivität wurde wie folgt berechnet:

Berechnung: 1 Unit = 1µmol Substratumsatz/min
Lambert-Beersches Gesetz

Abnahme des NADPH wurde bei 340 nm verfolgt (siehe enzymatischer Testansatz) = $\Delta E/min$

N = Verdünnungsfaktor Enzym
V = Volumen Enzym im ml (0,01)
$K_{küvette}$ = Küvettenvolumen = 1 ml
d = Schichtdicke der Küvette = 1 cm
$e_{NADPH}$= Extinktionskoeffizient NADPH = 6,22[$mM^{-1} * cm^{-1}$]

$$\text{Aktivität} = (\Delta E/min * N * V_{küvette})/(e_{NADPH} * V * d)$$

Proteinbestimmung wurde nach Bradford angewandt (Bio-Rad- Laboratories GmbH, Protein Assay)

Beispiel 3

Enzymkatalysierte Herstellung von Ethyl (S)-4 chloro-3-hydroxybutyrat

A.) im 5 Liter Maßstab

Für die enzymkatalysierte Synthese von Ethyl (S)-4 chloro-3-hydroxybutyrat aus 4-Chlor-3-oxo- butansäureethylester wurde der in Beispiel 2 gewonnene Rohextrakt der Alkohol-Dehydrogenase und das Coenzym NADP eingesetzt. Das oxidierte Coenzym wurde durch gleichzeitige Anwesenheit von Isopropanol kontinuierlich regeneriert, so dass die Reaktion nur katalytische Mengen an Coenzym erfordert.

Der Ansatz enthielt :

2 l Triethanolamin- Puffer 100mM pH =7.0, 1mM $MgCl_2$, 10% Glycerin,
400 mg NADP,
600 ml Isopropanol,
800 ml Essigsäureethylester,
600 ml 4-Chlor-3-oxo- butansäureethylester und
etwa 100 000 Units Alkohol-Dehydrogenase.

Nach 3 Tagen Rühren bei Raumtemperatur konnte gaschromatographisch der vollständige Umsatz des 4-Chlor-3-oxo-butansäureethylester zum Ethyl (S)-4 chloro-3-hydroxybutyrat mit über 99,9 % Enantiomerenreinheit nachgewiesen werden.

Nach Abtrennung der wässrigen Phase, Abdampfen des Lösungsmittels und gegebenenfalls Destillation erhält man das saubere Ethyl (S)-4 chloro-3-hydroxybutyrat mit über 99,9%iger Enantiomerenreinheit.

B.) im 501 Maßstab

Der Reaktionsansatz zum Umsatz von 10 l 4-Chlor-3-oxo- butansäureethylester ist wie folgt zusammengesetzt;:

18 l Triethanolamin- Puffer 100mM pH =7.0, 1mM $MgCl_2$, 10% Glycerin,
4 g NADP,
10 Isopropanol,
10 l Essigsäureethylester,
10l 4-Chlor-3-oxo- butansäureethylester und
etwa 2 Millionen Units Alkohol-Dehydrogenase (1,25l Rohextrakt).

Nach 7 Tagen Rühren bei Raumtemperatur konnte gaschromatographisch der vollständige Umsatz des 4-Chlor-3-oxo-butansäureethylesters zum Ethyl (S)-4 chloro-3-hydroxybutyrat mit über 99,9 % Enantiomerenreinheit nachgewiesen werden.

Beispiel 4

Biochemische Charakterisierung der klonierten Alkohol-Dehydrogenase aus Lactobacillus minor

A.) pH-Stabilität

Die Abhängigkeit der Aktivität des Enzyms bei Lagerung in Puffern mit verschiedenen pH-Werten wurde im Bereich von pH 4 bis 11 untersucht. Dazu wurden verschiedene Puffer (50mM) im Bereich von pH 4 bis 11 angesetzt und das in Beispiel 2 gereinigte Enzym darin 1:100 verdünnt und 30 min inkubiert. Alle Puffer enthielten 1 mM $MgCl_2$. Anschließend wurden davon 10µl im normalen Enzymtest eingesetzt (Triethanoiaminpuffer 100mM pH = 7.0, 1mM $MgCl_2$, 10 mM 4-Chlor-3-oxo- butansäureethylester und 0,19mM NADPH). Die Reaktion wurde für 1 min bei 30 °C und 340 nm verfolgt.

Ausgangswert ist dabei der Messwert, den man unmittelbar nach Verdünnung des Enzyms in Triethanolaminpuffer 50 mM pH = 7.0 erhält. Dieser Wert entsprach unter vorgegeben Bedingungen einer Extinktionsänderung von 0,20 /min und wurde als 100%-Wert gesetzt und alle folgenden Messwerte wurden zu diesem Wert ins Verhältnis gesetzt.

Tabelle 2

| PH-Wert | Puffersystem | Aktivität in % (n=2) | Puffersystem | Aktivität in % (n=2) |
|---|---|---|---|---|
| 4 | Na-acetat/ Essigsäure | 87,5 ± 6,5 | | |
| 4,5 | Na-acetat/ Essigsäure | 94,5 ± 3,0 | | |
| 5 | Na-acetat/ Essigsäure | 94,5 ± 1,5 | MES/NaOH | 55 ± 5 |
| 5,5 | $KH_2PO_4/K_2PO_4$ | 96 ± 3 | MES/NaOH | 77,1 ± 2,1 |
| 6 | $KH_2PO_4/K_2PO_4$ | 100 ± 0 | Triethanolamin/NaOH | 100 ± 0 |
| 6,5 | $KH_2PO_4/K_2PO_4$ | 97,5 ± 2,5 | Triethanolamin/NaOH | 100 ± 0 |
| 7 | $KH_2PO_4/K_2PO_4$ | 100 ± 0 | Triethanolamin/NaOH | 97,9 ± 2,1 |
| 7,5 | $KH_2PO_4/K_2PO_4$ | 97,5 ± 7,5 | Tris/HCl | 94,6 ± 1,3 |
| 8 | $KH_2PO_4/K_2PO_4$ | 93,0 ± 3,0 | Tris/HCl | 89,2 ± 0 |
| 8,5 | $KH_2PO_4/K_2PO_4$ | 102,5 ± 2,5 | Tris/HCl | 60 ± 4,2 |
| 9 | Glycin/NaOH | 76,5 ±1,5 | Tris/HCl | 63,1 ± 4,8 |
| 9,5 | Glycin/NaOH | 52,5 ± 7,5 | | |
| 10 | Glycin/NaOH | 52,5 ± 7,5 | | |
| 11 | Glycin/NaOH | 0,0 ± 0 | | |

Tabelle 2 zeigt, dass das Enzym eine gute pH-Stabilität insbesondere im sauren Bereich aufweist, dabei scheint die Enzymstabilität nicht nur vom pH-Wert, sondern auch vom verwendeten Puffersystem abhängig zu sein. Beispielsweise stellt man bei der Verwendung von TRIS und MES-Puffer bei gleichem pH-Wert eine stärkere Inaktivierung des Enzyms fest als im KPi-Puffer. Im KPi-Puffer zeigte sich im pH-Bereich von 5,5 bis 8,5 keine signifikante Inaktivierung.

B.) Temperaturstabilität

In analoger Weise wie unter A.) beschrieben wurde die Temperaturstabilität für den Bereich von 25 °C bis 50 °C bestimmt. Dazu wurde jeweils eine 1:100 Verdünnung des gereinigten Enzyms für 30 min bei der jeweiligen Temperatur inkubiert und anschließend bei 30 °C mit dem obigen Testansatz gemessen. Auch hier wurde als Ausgangswert der Meßwert herangezogen, den man unmittelbar nach Verdünnung des Enzyms in Triethanolaminpuffer 50 mM pH = 7.0 erhält. Dieser Wert wurde auch hier als 100%-Wert gesetzt. Die Alkohol-Dehydrogenase aus L. minor ist bis zu einer Temperatur von 40 °C stabil. Danach sinkt die Aktivität rapide ab.

Tabelle 3

| Temperatur | Aktivität in % (n=4) | Temperatur | Aktivität in % (n=4) |
|---|---|---|---|
| 25 | 101 ± 3,2 | 40 | 33,4 ± 3,8 |
| 30 | 81,2 ± 5,8 | 42 | 0 ±0 |
| 35 | 67,0 ± 1,6 | 45 | 0 ± 0 |
| 37 | 20,2 ± 2,4 | 50 | 0 ± 0 |

C.) pH-Optimum

Zur Bestimmung des pH-Optimums wurde die enzymatische Reaktion in dem jeweiligen in Tabelle 3 aufgeführten Puffer bestimmt. Die Konzentration des 4-Chlor-3-oxo- butansäureethylester betrug wie im Standardtest 10 mM und von NADPH 0,19 mM. Die Reaktion wurde bei 30 °C bestimmt. Dabei konnte für das erfindungsgemäße Enzym ein pH-Optimum zwischen 7 und 7,5 ermittelt werden.

Tabelle 4

| pH-Wert | Puffersystem | Aktivität in U/ml unverdünntes Enzym |
|---|---|---|
| 4 | Na-acetat/Essigsäure | 85 |
| 4,5 | Na-acetat/Essigsäure | 132 |
| 5 | MES/NaOH | 218 |
| 5,5 | MES/NaOH | 240 |
| 6 | Triethanolamin/NaOH | 381 |
| 6,5 | Triethanolamin/NaOH | 349 |
| 7 | Triethanolamin/NaOH | 510 |
| 7,5 | Tris/HCl | 707 |
| 8 | Tris/HCl | 585 |
| 8,5 | Tris/HCl | 486 |
| 9 | Tris/HCl | 488 |
| 10 | Glycin/NaOH | 131 |
| 11 | Glycin/NaOH | 0 |

D.) Temperatur-Optimum

Zur Bestimmung der optimalen Testtemperatur wurde die Enzymaktivität von 25 °C bis 60 °C gemessen. Der Testansatz entsprach der Standardkonzentration von 4-Chlor-3-oxo- butansäureethylester und NADPH. Wie aus Tabelle 5 ersichtlich hat das Enzym seine optimale Testtemperatur bei 55 °C, anschließend sinkt die Aktivität rapide ab.

Tabelle 5

| Temperatur | Aktivität in U/ml unverdünntes Enzym | Temperatur | Aktivität in U/ml unverdünntes Enzym |
|---|---|---|---|
| 25 | 540 | 45 | 2469 |
| 30 | 1235 | 50 | 2469 |
| 35 | 1968 | 55 | 2855 |
| 40 | 1621 | 60 | 0 |

E.) Substratspektrum

Ferner wurden anstelle von 4-Chlor-3-oxo-butansäureethylester noch weitere Substrate in dem enzymatischen Testansatz eingesetzt. Dafür wurde folgender Testansatz verwendet:

970 µl Triethanolaminpuffer (100 mM, pH = 7.0, 1 mM $MgCl_2$ mit 10mM Ketoverbindung)
20 µl NADPH (0,19mM im Testansatz)
10 µl Enzym (1:100)

Dabei wurde die mit 4-Chlor-3-oxo- butansäureethylester ermittelte Aktivität 100% gesetzt und die Enzymaktivitäten der anderen Substrate zu diesem Wert ins Verhältnis gesetzt.

Tabelle 6

| Substrat | Aktivität in % (n=2) |
|---|---|
| 4-Chlor-3-oxo- butansäureethylester | 100 |
| Ethylpyruvat | 192,3 ± 11,5 |
| 2-Octanon | 90,8 ± 1,2 |
| Acetoacetatmethylester | 120 ± 7,7 |
| 2-Oxo-4-phenylbutyratethylester | 62,7 ± 4,8 |

F.) Enzymstabilität in organischen Lösungsmitteln

Zur Untersuchung der Enzymstabilität bei Kontakt mit organischen Lösungsmitteln wurde die Alkohol-Dehydrogenase aus L. minor in den angegebenen Löungsmittelgemischen 1: 100 verdünnt und bei Raumtemperatur inkubiert (bei nicht-wassermischbaren organischen Lösungsmitteln bezieht sich die Verdünnung auf die wäßrige Phase). Dabei wurde eine ständige Durchmischung beider Phasen gewährleistet (Shaker, 200rpm). Anschließend wurden 10 µl der Enzymlösung im Standardtestansatz eingesetzt. Auch hier wurde der Ausgangswert nach Verdünnung im Puffer (Triethanolaminpuffer 100mM, pH = 7.0, 1mM $MgCl_2$) gleich 100 % gesetzt und alle weiteren Werte zu diesem ins Verhältnis gesetzt.

Tabelle 7:

| A.) wassermischbare Lösungsmittel: | | | | | |
|---|---|---|---|---|---|
| **Lösungsmittel** | **logP** | **t= 2h** | **T= 8h** | **t= 24h** | **t= 48h** |
| **Puffer** | | ***86*** | ***70*** | ***3*** | ***0*** |
| **10 % Isopropanol** | 0,28 | 32 | 34 | 16 | 0 |
| **20 % Isopropanol** | 0,28 | 16 | 17 | 7 | 0 |
| **10% DMSO** | -1,3 | 73 | 54 | 60 | 40 |
| **20% DMSO** | -1,3 | 73 | 54 | 57 | 40 |
| **1 M Sorbitol** | | 93 | 74 | 60 | 6 |
| **10 % Glyzerin** | -3,0 | 120 | 64 | 62 | 28 |
| **20% Glyzerin** | -3,0 | 120 | 100 | 100 | 104 |

Wie aus Tabelle 7A ersichtlich wirken Glyzerin, DMSO und Sorbitol aktivierend bzw. stabilisierend auf die eingesetzte Alkoholdehydrogenase. Das im Prozess einzusetzende Isopropanol hingegen wirkt inaktivierend.

| B.) nicht wassermischbare Lösungsmittel | | | | | |
|---|---|---|---|---|---|
| **Lösungsmittel** | **LogP** | **t= 2h** | **t= 8h** | **t= 24h** | **t= 48h** |
| **Puffer** | | ***86*** | ***70*** | ***3*** | ***0*** |
| **20% Essigsäureethylester** | 0,68 | 87 | 50 | 10 | 8 |
| **20 % Diethylether** | 0,85 | 53 | 42 | 37 | 23 |

(fortgesetzt)

| B.) nicht wassermischbare Lösungsmittel | | | | | |
|---|---|---|---|---|---|
| **Lösungsmittel** | **LogP** | **t= 2h** | **t= 8h** | **t= 24h** | **t= 48h** |
| **20 %Tert-Buthylmethylether** | 1,21 | 67 | 51 | 38 | 24 |
| **20 Diisopropylether** | 1,55 | 100 | 57 | 41 | 29 |
| **20 % Dibutylether** | 2,9 | 92 | 71 | 23 | 6 |
| **20 % Pentan** | 3,0 | 74 | 55 | 7 | 6 |
| **20 % Hexan** | 3,5 | 80 | 39 | 2 | 5 |
| **20 % Heptan** | 4 | 51 | 49 | 7 | 6 |
| **20 % Octan** | 4,5 | 87 | 47 | 2 | 1 |

Wie aus Tabelle 7B ersichtlich zeigt die untersuchte Alkoholdehydrogenase in einer breiten Zahl organischer Lösungsmittel eine beachtliche Stabilität. Auffallend ist dabei, dass Lösungsmittel mit logP- Werten zwischen 0 und 3 die untersuchte Alkoholdehydrogenase nicht stärker inhibieren als solche mit log P-Werten zwischen 3 und 4,5, insbesondere im Hinblick auf längere Inkubationszeiten (24 h und 48 h) haben Lösungsmittel mit log P-Werten zwischen 0 und 3 stabilisierende Wirkung auf die untersuchte ADH, verglichen mit den entsprechenden Werten im Puffer. Die untersuchten aliphätischen Lösungsmittel Pentan, Hexan, Heptan und Octan zeigen diese stabilisierende Wirkung bei Langzeitinkubation nicht.

Der log P-Wert einer KomponenteX ist der Logarithmus des Verteilungskoeffizient von X im Octanol/Wasser Zweiphasensystem (50/50)

P = Konzentration von X in Octanolphase/ Konzentration von X in wäßriger Phase

G. Enzymstabilität unter Prozessbedingungen

Zur Untersuchung der Enzymstabilität unter Prozessbedingungen wurde die Alkohol-Dehydrogenase aus L. minor mit den im Zwei-Phasen-System verwendeten Löungsmittelgemischen 1 : 100 verdünnt und bei Raumtemperatur inkubiert. Anschließend wurden 10 µl der Enzymlösung im Standardtestansatz eingesetzt.

In Tabelle 8 sind die Enzymaktivitäten in % vom Ausgangswert dargestellt.

Tabelle 8

| | 6 h | 20 h | 46 h | 60 h | 84 h |
|---|---|---|---|---|---|
| Triethanolamin-Puffer, 100 mM, 1 mM $MgCl_2$ | 100 | 75 | 0 | 0 | 0 |
| Mischung B | 100 | 85 | 80 | 60 | 55 |
| Mischung C | 110 | 95 | 95 | 85 | 80 |
| Mischung D | 100 | 65 | 55 | 50 | 50 |
| Mischung B: Puffer, 10% Glycerin, 10% Isopropanol<br>Mischung C: Puffer, 20% Glycerin, 10% Isopropanol<br>Mischung D: Puffer, 10% Glycerin, 10% Isopropanol + 20% Essigsäureethylester | | | | | |

Es wurde festgestellt, dass die rekombinate Alkohol-Dehydrogenase aus L. minor in der im Zwei-Phasen-System verwendeten Kombination von Lösungsmitteln mehrere Tage stabil und aktiv ist.

## Patentansprüche

**1.** Verfahren zur enantioselektiven Reduktion einer Ketoverbindung der Formel I

$R^1$-C(O)-$R^2$ (I)

wobei $R^1$ und $R^2$ unabhängig voneinander gleich oder verschieden sind und für

1. Wasserstoffatom,
2. -($C_1$-$C_{20}$)-Alkyl, worin Alkyl geradkettig oder verzweigt ist,
3. -($C_2$-$C_{20}$)-Alkenyl, worin Alkenyl geradkettig oder verzweigt ist und gegebenenfalls ein, zwei, drei oder vier Doppelbindungen enthält,
4. -($C_2$-$C_{20}$)-Alkinyl, worin Alkinyl geradkettig oder verzweigt ist und gegebenenfalls ein, zwei, drei oder vier Dreifachbindungen enthält,
5. -($C_6$-$C_{14}$)-Aryl,
6. -($C_1$-$C_8$)-Alkyl-($C_6$-$C_{14}$)-Aryl, oder
7. $R^1$ und $R^2$ bilden zusammen mit dem -C(O)-Rest ein -($C_6$-$C_{14}$)-Aryl oder einen -($C_5$-$C_{14}$)-Heterocydus, wobei die oben unter 1. bis 7. genannten Reste unsubstituiert sind oder ein- bis dreifach substituiert sind unabhängig voneinander durch

   a) -OH,
   b) Halogen, wie Fluor, Chlor, Brom oder Jod,
   c) -$NO_2$,
   d) -C(O)-O-($C_1$-$C_{20}$)-Alkyl, worin Alkyl gerade oder verzweigt ist und unsubstituiert oder ein- bis dreifach substituiert ist durch Halogen, Hydroxyl, Amino oder Nitro, oder
   e) -($C_5$-$C_{14}$)-Heterocyclus, der unsubstituiert oder ein- bis dreifach substituiert ist durch Halogen, Hydroxyl, Amino oder Nitro,

steht, **dadurch gekennzeichnet, dass**

a) eine Verbindung der Formel I mit einem Amteil von 2% bis 30%, bezogen auf das Gesamtvolumen des Reaktionsansatzes, Alkohol-dehydrogenase, Wasser, Cofaktor NADPH oder NADH und ein organisches Lösungsmittel mit einem logP von 0,5 bis 4,0;
b) in einem Zwei-Phasen-System aus Wasser, organischem Lösungsmittel und der Verbindung der Formel (I) inkubiert wird;
c) der durch die Alkoholdehydrogenase gebildete oxidierte Cofaktor kontinuierlich regeneriert, und
d) die chirale Hydroxyverbindung isoliert wird.

**2.** Verfahren gemäß Anspruch 1, **dadurch gekennzeichnet, dass** eine Verbindung der Formel (I) aus der Reihe 4-Chlor-3-oxo- butansäureethylester, Acetophenon, Acetessigsäuremethylester, Ethyl-2-oxo-4-phenylbutyrat, 2,5-Hexandion, Ethylpyruvat oder 2-Octanon eingesetzt wird.

**3.** Verfahren gemäß Anspruch 1 oder 2, **dadurch gekennzeichnet, dass** ein organisches Lösungsmittel mit einem logP von 0,6 bis 3,0, insbesondere von 0,6 bis 1,9, eingesetzt wird.

**4.** Verfahren gemäß Anspruch 3, **dadurch gekennzeichnet, dass** ein organisches Lösungsmittel mit einem logP von 0,63 bis 1,75 eingesetzt wird.

**5.** Verfahren gemäß Anspruch 1, **dadurch gekennzeichnet, dass** als organisches Lösungsmittel Diethylether, tertiär-Butylmethylether, Diisopropylether oder Essigsäureethylester eingesetzt wird.

**6.** Verfahren gemäß einem oder mehreren der Ansprüche 1 bis 5, **dadurch gekennzeichnet, dass** eine Alkoholdehydrogenase aus Hefe, Pferdeleber, Thermoanaerobium brockii, Rhodococcus erythropolis, Lactobacillus kefir, Lactobacillus brevis, Lactobacillus minor oder eine Alkoholdehydrogenase mit der Aminosäuresequenz gemäß SEQ ID NO: 4 eingesetzt wird.

**7.** Verfahren gemäß einem oder mehreren der Ansprüche 1 bis 6, **dadurch gekennzeichnet, dass** ein Puffer wie Kaliumphosphat-, Tris/HCl- oder Triethanolamin-Puffer mit einem pH-Wert von 5 bis 10, bevorzugt mit einem pH-Wert von 6 bis 9, zugesetzt wird.

8. Verfahren gemäß Anspruch 7, **dadurch gekennzeichnet, dass** dem Puffer Magnesiumionen wie $MgCl_2$ , in einer Konzentration von 0,2 mM bis 10 mM, bevorzugt 0,5 mM bis 2 mM, zugesetzt werden.

9. Verfahren gemäß einem oder mehreren der Ansprüche 1 bis 8, **dadurch gekennzeichnet, dass** als Cofaktor NADPH oder NADH in einer Menge von 0,05 mM bis 0,25 mM, insbesondere von 0,06 mM bis 0,2 mM, bezogen auf die wässrige Phase, zugesetzt wird.

10. Verfahren gemäß einem oder mehreren der Ansprüche 1 bis 9, **dadurch gekennzeichnet, dass** als Stabilisator für die Alkoholdehydrogenase Glycerin, Sorbitol oder Dimethylsulfoxid zugefügt wird.

11. Verfahren gemäß einem oder mehreren der Ansprüche 1 bis 10, **dadurch gekennzeichnet, dass** Isopropanol zugefügt wird.

12. Verfahren gemäß einem oder mehreren der Ansprüche 1 bis 11, **dadurch gekennzeichnet, dass** die Verbindungen der Formel (I) in einer Menge von 5% bis 30 % bezogen auf das Gesamtvolumen, bevorzugt von 10% bis 25%, insbesondere von 15 % bis 22 %, eingesetzt werden.

13. Verfahren gemäß einem oder mehreren der Ansprüche 1 bis 12, **dadurch gekennzeichnet, dass** die Umsetzung bei einer Temperatur von etwa 10°C bis 70 °C, bevorzugt von 30 °C bis 60 °C, durchgeführt wird.

14. Verfahren gemäß einem oder mehreren der Ansprüche 1 bis 13, **dadurch gekennzeichnet, dass** das organische Lösungsmittel in einer Menge von 1 % bis 90%, bezogen auf das Gesamtvolumen des Reaktionsansatzes, vorzugsweise von 15% bis 60%, insbesondere von 20% bis 50% eingesetzt werden.

15. Verfahren gemäß einem oder mehreren der Ansprüche 1 bis 14, **dadurch gekennzeichnet, dass** das Verhältnis von organischen Lösungsmittel zu Wasser von 9 zu 1 bis 1 zu 9, vorzugsweise von 1 zu 1 bis 1 zu 3, beträgt.

16. Verfahren gemäß Anspruch 10, **dadurch gekennzeichnet, dass** der Stabilisator in einer Menge von 5% bis 30% bezogen auf das Volumen des gesamten Reaktionsansatzes, bevorzugt von 10% bis 20%, insbesondere 20%, eingesetzt wird.

17. Verfahren gemäß Anspruch 11, **dadurch gekennzeichnet, dass** Isopropanol in einer Menge von 5% bis 30% bezogen auf das Volumen des gesamten Reaktionsansatzes, bevorzugt von 10% bis 20%, insbesondere 10%, eingesetzt wird.

18. Verfahren gemäß Anspruch 6, **dadurch gekennzeichnet, dass** die Alkohol-dehydrogenase in einer Menge von 20 000 U bis 200 000 U pro kg umzusetzender Verbindung der Formel (I), bevorzugt etwa 100 000 U, eingesetzt wird.

Europäisches Patentamt
European Patent Office
Office européen des brevets

## EUROPÄISCHER RECHERCHENBERICHT

Nummer der Anmeldung
EP 09 01 0760

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 342 767 A (WONG CHI-HUEY ET AL) 30. August 1994 (1994-08-30)<br>* Spalte 5 *<br>* Spalte 9 - Spalte 13 *<br>* Spalte 19 - Spalte 21 *<br>* Ansprüche 1-6 * | 1-18 | INV.<br>C12N15/53<br>C12N9/04<br>C12N1/21<br>C12P7/02 |
| X | US 5 385 833 A (BRADSHAW CURT W [US] ET AL) 31. Januar 1995 (1995-01-31)<br>* das ganze Dokument * | 1-18 | |
| A,D | EP 0 796 914 A (BOEHRINGER MANNHEIM GMBH) 24. September 1997 (1997-09-24)<br>* Seite 2 - Seite 4 *<br>* Seite 16 - Seite 17 * | | |
| A | HUMMEL W: "NEW ALCOHOL DEHYDROGENASES FOR THE SYNTHESIS OF CHIRAL COMPOUNDS" ADVANCES IN BIOCHEMICAL ENGINEERING, BIOTECHNOLOGY, SPRINGER, BERLIN, DE, Bd. 58, 1997, Seiten 145-184, XP000677754 ISSN: 0724-6145<br>* Seite 164 - Seite 176 * | | RECHERCHIERTE SACHGEBIETE (IPC)<br>C12P<br>C12N |
| A | WO 96/38577 A (BIOTECHNOLOGY RES & DEV ;UNIV IOWA RES FOUND (US); DORDICK JONATHA) 5. Dezember 1996 (1996-12-05)<br>* Seite 8 * | | |
| A | BRANDSHAW C W ET AL: "ENZYMATIC SYNTHESIS OF (R) AND (S) 1-DEUTEROHEXANOL" APPLIED BIOCHEMISTRY AND BIOTECHNOLOGY, CLIFTON, NJ, US, Bd. 33, Nr. 1, 1. April 1992 (1992-04-01), Seiten 15-24, XP002053169 ISSN: 0273-2289<br>* Seite 15 *<br>* Seite 22 * | | |
| | -/-- | | |

Der vorliegende Recherchenbericht wurde für alle Patentansprüche erstellt

1

| Recherchenort | Abschlußdatum der Recherche | Prüfer |
|---|---|---|
| München | 6. Oktober 2009 | Schneider, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 09 01 0760

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A,D | BRUCE L J ET AL: "SOLVENT SELECTION STRATEGIES FOR EXTRACTIVE BIOCATALYSIS" BIOTECHNOLOGY PROGRESS, Bd. 7, Nr. 2, 1991, Seiten 116-124, XP002237300 ISSN: 8756-7938 * das ganze Dokument * ----- | | |
| A | JONSSON ASA ET AL: "Thermodynamic and kinetic aspects on water vs. organic solvent as reaction media in the enzyme-catalysed reduction of ketones." BIOCHIMICA ET BIOPHYSICA ACTA, Bd. 1430, Nr. 2, 19. März 1999 (1999-03-19), Seiten 313-322, XP002237301 ISSN: 0006-3002 * das ganze Dokument * ----- | | |
| A | HUMMEL WERNER: "Large-scale applications of NAD(P)-dependent oxidoreductases: Recent developments." TRENDS IN BIOTECHNOLOGY, Bd. 17, Nr. 12, Dezember 1999 (1999-12), Seiten 487-492, XP002237302 ISSN: 0167-7799 * das ganze Dokument * ----- | | RECHERCHIERTE SACHGEBIETE (IPC) |

1

Der vorliegende Recherchenbericht wurde für alle Patentansprüche erstellt

| Recherchenort | Abschlußdatum der Recherche | Prüfer |
|---|---|---|
| München | 6. Oktober 2009 | Schneider, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 09 01 0760

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-10-2009

| Im Recherchenbericht angeführtes Patentdokument | Datum der Veröffentlichung | Mitglied(er) der Patentfamilie | Datum der Veröffentlichung |
|---|---|---|---|
| US 5342767 A | 30-08-1994 | US 5225339 A | 06-07-1993 |
| US 5385833 A | 31-01-1995 | KEINE | |
| EP 0796914 A | 24-09-1997 | DE 19610984 A1 | 25-09-1997 |
| | | ES 2201216 T3 | 16-03-2004 |
| | | JP 3950196 B2 | 25-07-2007 |
| | | JP 10028590 A | 03-02-1998 |
| | | US 6037158 A | 14-03-2000 |
| WO 9638577 A | 05-12-1996 | AU 704262 B2 | 15-04-1999 |
| | | AU 5979396 A | 18-12-1996 |
| | | CA 2222733 A1 | 05-12-1996 |
| | | EP 0828847 A1 | 18-03-1998 |
| | | IL 122277 A | 31-08-2000 |
| | | JP 11506333 T | 08-06-1999 |
| | | US 5719039 A | 17-02-1998 |
| | | US 6171813 B1 | 09-01-2001 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4014573
- DE 19610984


### In der Beschreibung aufgeführte Nicht-Patentliteratur


- **M.R. Kula ; U. Kragel.** Dehydrogenases in Synthesis of Chiral Compounds
- **R. N. Patel.** Stereoselective Biocatalyses. 2000
- Dehydrogenases-Characteristics, Design of Reaction Conditions, and Application. **Peters J. ; H.J. Rehm ; G. Reed.** Biotechnology, Vol 3, Bioprocessing. VCH Weinheim, 1993, vol. 3
- **J. Lynda et al.** Solvent selection strategies for extractive Biocatalysis. *Biotechnol. Prog.,* 1991, vol. 7, 116-124

SEQUENCE LISTING

<110> IEP GmbH

<120> Enzymatisches Verfahren zur enantioselektiven Reduktion von Ketoverbindungen

<130> I 12898

<160> 4

<170> PatentIn version 3.3

<210> 1
<211> 795
<212> DNA
<213> Artificial

<220>
<223> Core Sequence from Lactobacillus sp. with Histag

<400> 1
atgagaggat cgcatcacca tcaccatcac ggatccatga ccgatcggtt gaaggggaaa 60

gtagcaattg taactggcgg taccttggga attggcttgg caatcgctga taagtttgtt 120

gaagaaggcg caaaggttgt tattaccggc cgtcacgctg atgtaggtga aaaagctgcc 180

agatcaatcg gcggcacaga cgttatccgt tttgtccaac acgatgcttc tgatgaaacc 240

ggctggacta agttgtttga tacgactgaa gaagcatttg gcccagttac cacggttgtc 300

aacaatgccg gaattgcggt cagcaagagt gttgaagata ccacaactga agaatggcgc 360

aagctgctct cagttaactt ggatggtgtc ttcttcggta cccgtcttgg aatccaacgt 420

atgaagaata aaggactcgg agcatcaatc atcaatatgt catctatcga aggttttgtt 480

ggtgatccag ctctgggtgc atacaacgct tcaaaaggtg ctgtcagaat tatgtctaaa 540

tcagctgcct tggattgcgc tttgaaggac tacgatgttc gggttaacac tgttcatcca 600

ggttatatca agacaccatt ggttgacgat cttgaagggg cagaagaaat gatgtcacag 660

cggaccaaga caccaatggg tcatatcggt gaacctaacg atatcgcttg gatctgtgtt 720

tacctggcat ctgacgaatc taaatttgcc actggtgcag aattcgttgt cgacggaggg 780

tacaccgccc aatag 795

<210> 2
<211> 37
<212> DNA
<213> Artificial

<220>
<223> DNA Oligonucleotide

<220>
<221> misc_feature
<222> (14)..(14)
<223> n is a, c, g, or t

<220>
<221> misc_feature
<222> (20)..(20)

<223> n is a, c, g, or t

<220>
<221> misc_feature
<222> (29)..(29)
<223> n is a, c, g, or t

<220>
<221> misc_feature
<222> (35)..(35)
<223> n is a, c, g, or t

<400> 2
gcggatccat gacngaycgn ttraarggna argtngc 37

<210> 3
<211> 36
<212> DNA
<213> Artificial

<220>
<223> DNA Oligonucleotide

<220>
<221> misc_feature
<222> (16)..(16)
<223> n is a, c, g, or t

<220>
<221> misc_feature
<222> (19)..(19)
<223> n is a, c, g, or t

<220>
<221> misc_feature
<222> (25)..(25)
<223> n is a, c, g, or t

<220>
<221> misc_feature
<222> (28)..(28)
<223> n is a, c, g, or t

<220>
<221> misc_feature
<222> (34)..(34)
<223> n is a, c, g, or t

<400> 3
gggaagcttc taytgngcng trtanccncc rtcnac 36

<210> 4
<211> 264
<212> PRT
<213> Artificial

<220>
<223> Histidine Taged Lactobacillus sp. Protein

<400> 4

Met Arg Gly Ser His His His His His His Gly Ser Met Thr Asp Arg
1 5 10 15

```
Leu Lys Gly Lys Val Ala Ile Val Thr Gly Gly Thr Leu Gly Ile Gly
            20                  25                  30

Leu Ala Ile Ala Asp Lys Phe Val Glu Glu Gly Ala Lys Val Val Ile
        35                  40                  45

Thr Gly Arg His Ala Asp Val Gly Glu Lys Ala Ala Arg Ser Ile Gly
    50                  55                  60

Gly Thr Asp Val Ile Arg Phe Val Gln His Asp Ala Ser Asp Glu Thr
65                  70                  75                  80

Gly Trp Thr Lys Leu Phe Asp Thr Thr Glu Glu Ala Phe Gly Pro Val
                85                  90                  95

Thr Thr Val Val Asn Asn Ala Gly Ile Ala Val Ser Lys Ser Val Glu
            100                 105                 110

Asp Thr Thr Thr Glu Glu Trp Arg Lys Leu Leu Ser Val Asn Leu Asp
        115                 120                 125

Gly Val Phe Phe Gly Thr Arg Leu Gly Ile Gln Arg Met Lys Asn Lys
    130                 135                 140

Gly Leu Gly Ala Ser Ile Ile Asn Met Ser Ser Ile Glu Gly Phe Val
145                 150                 155                 160

Gly Asp Pro Ala Leu Gly Ala Tyr Asn Ala Ser Lys Gly Ala Val Arg
                165                 170                 175

Ile Met Ser Lys Ser Ala Ala Leu Asp Cys Ala Leu Lys Asp Tyr Asp
            180                 185                 190

Val Arg Val Asn Thr Val His Pro Gly Tyr Ile Lys Thr Pro Leu Val
        195                 200                 205

Asp Asp Leu Glu Gly Ala Glu Glu Met Met Ser Gln Arg Thr Lys Thr
    210                 215                 220

Pro Met Gly His Ile Gly Glu Pro Asn Asp Ile Ala Trp Ile Cys Val
225                 230                 235                 240

Tyr Leu Ala Ser Asp Glu Ser Lys Phe Ala Thr Gly Ala Glu Phe Val
                245                 250                 255

Val Asp Gly Gly Tyr Thr Ala Gln
            260
```